# EUROPÄISCHE PATENTSCHRIFT

(11) **EP 0 849 221 B1**
(45) Veröffentlichungstag und Bekanntmachung des Hinweises auf die Patenterteilung: **03.01.2001**
(21) Anmeldenummer: 97810960.1
(22) Anmeldetag: 10.12.1997
(51) Int. Cl.: C01B 39/02, C09C 1/40, C09B 63/00, C08K 3/00, C03C 4/02, C04B 33/14, A61K 7/00, C09D 7/12, C09D 11/00

(54) **Farbmittelbeladenes Molekularsieb**
Dye-containing molecular sieve
Tamis moléculaire contenant un colorant

(30) Priorität: 19.12.1996 CH 312396
(43) Veröffentlichungstag der Anmeldung: 24.06.1998
(73) Patentinhaber: Ciba Specialty Chemicals Holding Inc., 4057 Basel (CH)
(72) Erfinder: Hölderich, Wolfgang, 67227 Frankenthal (DE); Röhrlich, Nadja, 52080 Aachen (DE); Chassot, Laurent, 1724 Praroman (CH)

(56) Entgegenhaltungen:
- WO-A-93/17965
- WO-A-93/17966
- WO-A-95/19398
- DE-A- 4 126 461
- YOON-MYEONG JIN AND HAZKE CHON: "A novel method for encapsulation of dyes into ALPO4-8 molecular sieve" CHEMICAL COMMUNICATIONS., Nr. 2, 21.Januar 1996, CIETY OF CHEMISTRY GB, Seiten 135-136, XP002060573
- MARIA LUZ CANO & AL.: "Triarylmethylium cations encapsulated within zeolite supercages" JOURNAL OF THE AMERICAN CHEMICAL SOCIETY., Bd. 118, 1996, DC US, Seiten 11006-11013, XP002060575
- F. RAMOA RIBEIRO: "Adaptation of the porosity of zeolites for shape selective reactions" CATALYSIS LETTERS., Bd. 22, Nr. 1,2, November 1993, BASEL CH, Seiten 107-121, XP002060574

## Beschreibung

Die vorliegende Erfindung betrifft ein Molekularsieb, enthaltend in allen oder nur teilweise allen seinen Hohlräumen Farbmittelmoleküle sowie ein mit ihm kovalent verbundenes, seine Porendurchmesser verkleinerndes Modifizierungsmittel.

Des weiteren betrifft die Erfindung ein Verfahren zur Herstellung des erfindungsgemäßen Molekularsiebes sowie dessen Verwendung als Pigment zur Einfärbung von hochmolekularen organischen Materialien, insbesondere Biopolymeren und Kunststoffen, Gläsern, keramischen Produkten, für Zubereitungen in der dekorativen Kosmetik, zur Herstellung von Lacken, insbesondere Automobillacken, Druckfarben, Tinten, Dispersionsfarben und Farbfiltern sowie die erfindungsgemässen Molekularsiebe enthaltende Materialien.

An Molekularsiebe adsorbierte Farbstoffe sind seit längerem bekannt (siehe DE-A 41 26 461, S.2, Z. 5-21). Dabei ist in der Regel die Wechselwirkung zwischen den Farbstoffen und den Molekularsieben so stark, daß beim Erhitzen eine Desorption des Farbstoffes nur selten zu beobachten ist. Andererseits kann es bei geeigneten Lösungsmitteln zu einer Desorption kommen, wobei adsorbierte Farbstoffmoleküle vom Molekularsieb durch Auslaugen ("leaching") entfernt werden.

In der US 4,018,870 wird die Synthese siliciumhaltiger Molekularsiebe in Gegenwart von Methylenblau und Acriflavin, beides wasserlösliche, basische Farbstoffe, als Template beschrieben. Bei dieser sogenannten Templatsynthese wird das Molekularsieb in Gegenwart von einer als molekulare Schablone dienenden Verbindung - einem sogenannten Templatsynthetisiert. Die Template, in diesem Falle die Farbstoffmoleküle, werden auf diese Weise in die sich bildenden Hohlräume des entsprechenden Molekularsiebes eingebaut. Nach diesem Prinzip sind je nach Templat spezielle Molekularsiebe zugänglich. Um Molekularsiebe mit freien Poren und Hohlräumen zu erhalten, werden die in den Hohlräumen der erhaltenen Molekularsiebe eingeschlossenen organischen Templatverbindungen thermisch zersetzt.

Um den Nachteil des Auslaugens zu beseitigen, wurde daher in der DE-A 41 26 461, in Anlehnung an das bereits zitierte US-Dokument, vorgeschlagen, Farbstoffmoleküle in das Gerüst der Molekularsiebe einzubauen. Speziell werden in der DE-A 41 26 461 farbstoffbeladene anorganische Molekularsiebe beschrieben, in denen ein wasserunlöslicher organischer Farbstoff irreversibel in die Hohlraumstruktur des Molekularsiebes eingebaut ist. Die in der DE-A 41 26 461 beschriebenen Molekularsiebe sind durch Templatsynthese zugänglich. Voraussetzungen für den irreversiblen Einbau der Farbstoffmoleküle sind Molekülgrößen der Farbstoffmoleküle, die nicht größer als höchstens die Größe der Hohlräume des Molekularsiebes und größer als dessen freier Porendurchmesser sind.

In Zeolites 4 (1984), S. 30-34 wird die sogenannte "ship-in-the-bottle"-Synthese von Kobalt-, Nickel- und Kupfer-Phthalocyaninen ("Pc") im Zeolith Faujasit beschrieben. Bei dieser Verfahrensweise werden zunächst die im Zeolithen vorhanden Kationen gegen die genannten Übergangsmetallkationen ausgetauscht. In einem anschließenden Schritt wird dann durch Zugabe von ortho-Phthalodinitril der entsprechende Pc-Komplex unter anderem in den Hohlräumen des Faujasits gebildet. Die in den Hohlräumen gebildeten Pc-Komplexe diffundieren in der Regel aus sterischen Gründen nicht aus den Hohlräumen hinaus. Bei einigen Anwendungen ist dies jedoch - unerwünschterweise - der Fall.

Nachteilig an den in der DE-A 41 26 461, der US 4,018,870 und in Zeolites 4 (1984) beschriebenen Molekularsieben ist die durch die Molekülgrößen der Farbstoffe in Verbindung mit den Hohlraumvolumina und freien Porendurchmessern begrenzte Zahl an möglichen Farbstoff-Molekularsieb-Kombinationen, in denen Farbstoffmoleküle irreversibel im Molekularsieb festgehalten werden bzw. festgehalten werden sollen. Im Falle der DE-A 41 26 461 kommt noch hinzu, daß man Amine wie Triethanolamin als Template zugeben muß. Die Template verbleiben jedoch teilweise in den Molekularsieben und verhindern dadurch eine vollständige Beladung des Molekularsiebes mit dem Farbstoff. Schließlich werden auch Farbstoffe wie Thioindigo genannt, die wegen ihrer Molekülgröße durchaus aus den Poren der Molekularsiebe hinausdiffundieren können.

Die Veränderung der Oberfläche, insbesondere der Porendurchmesser, von Molekularsieben ist mehrfach in der Literatur beschrieben worden:

Murakami et al. beschreiben in "Acid-Base Catalysis", Proceedings of the International Symposium on Acid Base Catalysis Sapporo, (November 28 - December 1, 1988), VCH und Kodansha, edited by Tanabe, Hattori, Yamaguchi und Tanaka, S. 255 - 266, den Einfluß der Porengöße auf die ,,shape selectivity" bei Crack-Reaktionen. Die Porengröße wird nach der von Murakami et al. beschriebenen Methode durch Behandlung der Zeolithe mit Siliconalkoxiden verändert.

Die Veränderung der Porendurchmesser von Zeolithen mit Metallchloriden wie SiCl₄, ZnCl₂, GaCl₃, GeCl₄, TiCl₄, SnCl₄ sowie Si(OMe)₄, Silan (SiH₄), Disilan (Si₂H₆), und Diboran (B₂H₆) beschreibt Ribeiro in Catalysis Letters 22 (1993), S.107-121. Insbesondere wird der Einfluß des Grades der Oberflächenbelegung in bezug auf die Ausbeute an para-Xylol bei der Alkylierung von Toluol mit Methanol diskutiert.

Detailliertere Untersuchungen über die Veränderung der Porendurchmesser an Zeolithen durch Silan und Diboran (J.Chem.Soc., Faraday Trans. 1, 1983,79, 2821-2834) sowie Disilan (J.Phys.Chem. 1990, 94, 2582-2586) beschreiben Vansant et al.

Benazzi et al. beschreiben in Microporous Materials, 2 (1994) S.251-259 die Porenverengung bei Zeolithen durch Behandlung mit verschiedenen TetraalkylzinnVerbindungen (SnR₄ (R=Me, Et, i-Pr, Ph und Cyclohexyl) sowie Bu₃SnH.

In der WO 92/21726 werden Pigmente mit zusammengesetzter Struktur beschrieben, wobei auf ein wasserunlösliches Kernmaterial mindestens eine Schicht aus einem wasserlöslichen Färbemittel adsorbiert ist, und das Färbemittel wiederum mit einer transparenten Schicht bedeckt ist. Als transparente Schichten werden Materialien aus Aluminiumoxid, Aluminiumsilikat und amorpher Kieselsäure, letzteres erhältlich aus Wasserglas oder einem Silikasol, genannt. Nachteilig an dieser Methode ist einerseits die Beschränkung auf nur wasserlösliche Färbemittel, und andererseits die für viele Anwendungen nicht ausreichende Haftung des Färbemittels durch Adsorbtion, insbesondere bei thermischer Belastung oder mechanischer Beanspruchung.

Aufgabe der vorliegenden Erfindung war es daher, farbmittelbeladene Molekularsiebe zur Verfügung zu stellen, die die oben genannten Nachteile nicht aufweisen. Insbesondere sollte das Auslaugen ("leaching"), das Ausbluten ("bleeding"), d.h. durch die lösende Wirkung von Weichmachern verursachte Migration der in Kunststoffen enthaltenden Farbmitteln in einen anderen, mit dem Kunststoff in Kontakt stehenden Stoff, die thermische Stabilität, die Resistenz gegenüber Lösungsmitteln, die Lichtstabilität, die chemische Resistenz verbessert sowie die Beladungshöhe des Molekularsiebes mit Farbmittel gesteigert werden.

Demgemäß wurde das eingangs definierte Molekularsieb, enthaltend in allen oder nur teilweise allen seinen Hohlräumen Farbmittelmoleküle sowie ein mit ihm kovalent verbundenes, seine Porendurchmesser verkleinerndes Modifizierungsmittel, gefunden.

Des weiteren wurden ein Verfahren zu dessen Herstellung, dessen Verwendung als Pigment zur Einfärbung von hochmolekularen organischen Materialien, insbesondere Biopolymeren und Kunststoffen, Gläsern, keramischen Produkten, für Zubereitungen in der dekorativen Kosmetik, zur Herstellung von Lacken, insbesondere Automobillacken, Druckfarben, Tinten, Dispersionsfarben und Farbfiltern sowie die erfindungsgemässen Molekularsiebe enthaltende Materialien gefunden.

Das erfindungsgemäße Molekularsieb enthält in allen oder teilweise allen seinen Hohlräumen Farbmittelmoleküle.

Des weiteren enthält das erfindungsgemäße Molekularsieb ein mit ihm kovalent verbundenes, seine Porendurchmesser verkleinerndes Modifizierungsmittel. Das Modifizierungsmittel reagiert nach bisherigen Beobachtungen bevorzugt mit den funktionellen Gruppen wie OH-Gruppen des Molekularsiebes, die sich an dessen äußeren Oberfläche befinden. Hierdurch kommt es auch zu einer Verkleinerung der Porendurchmesser, was indirekt daran zu erkennen ist, daß man modifizierte Molekularsiebe herstellen kann, die nicht auslaugen und/oder ausbluten. Man kann dies damit erklären, daß die Farbmittelmoleküle, die sich in den Hohlräumen eines solchermaßen modifizierten Molekularsiebes befinden, nicht mehr durch die nunmehr verengten Poren dringen können und mithin in den Hohlräumen eingeschlossen sind.

Man kann auch die Modifizierung, insbesondere durch Steuerung der Menge an Modifzierungsmittel und der Dauer der Reaktion mit dem Modifizierungsmittel, so vornehmen, daß beispielsweise nicht alle Poren verkleinert werden. So hergestellte Molekularsiebe können noch ausbluten oder auslaugen, jedoch in vermindertem Maße im Vergleich zu einem unmodifizierten Molekularsieb. Es ist somit möglich, je nach gewünschter Anwendung, die Modifizierung so vorzunehmen, daß man Molekularsiebe erhält, die mehr oder weniger ausbluten und/oder auslaugen, oder die nicht mehr ausblutenund/oder auslaugen. Es versteht sich von selbst, daß man bei Molekularsieben mit Mikroporen in der Regel weniger Modifizierungsmittel benötigt als bei mesoporösen Molekularsieben.

Die erfindungsgemäßen Molekularsiebe sind erhältlich durch (a) vollständiges oder teilweises Füllen deren Hohlräume mit Farbmittelmolekülen und anschließende Verkleinerung deren Porendurchmesser durch Reaktion mit einem Modifizierungsmittel, oder (b) Verkleinerung der Porendurchmesser eines in allen oder nur teilweise allen seinen Hohlräumen mit Farbmittelmolekülen gefüllten Molekularsiebes durch Reaktion mit einem Modifizierungsmittel.

Nach bisherigen Beobachtungen kann man als Modifizierungsmittel alle bekannten Verbindungen oder deren Gemische einsetzen, die in der Lage sind, kovalente Bindungen mit an der äußeren Oberfläche von Molekularsieben, einschließlich der nach außen gerichteten Porenöffnungen, befindlichen funktionellen Gruppen des Molekularsiebes einzugehen, insbesondere solche Verbindungen, die dabei die Porendurchmesser von Molekularsieben zu verkleinern vermögen.

Bevorzugt setzt man als Modifizierungsmittel mindestens eine Verbindung ein, die ausgewählt ist aus der Gruppe, bestehend aus Metallhalogeniden, Siliconalkoxiden, Kohlenstoff-Zinnverbindungen, Silicium-Wasserstoffverbindungen, Tetraalkylorthosilikaten, Trialkyl-, Dialkyl-, Monoalkyl- und Triarylchlorsilanen, Disiloxanen, Diboran, Silikatsole und - kolloide sowie halogenierte Polysiloxane.

Als Metallhalogenide, bevorzugt Metallchloride, kann man SiCl₄, ZnCl₂, GaCl₃, GeCl₄, TiCl₄, SnCl₄ wie beschrieben von Ribeiro in Catalysis Letters 22 (1993), S.107-121, einsetzen. Besonders bevorzugt ist Siliciumtetrachlorid.
Als Siliconalkoxiden kann man vorzugsweise Siliciumtetraalkoxide wie Si(OR)₄ (mit R = Me, Et, n-, i-Pr, n-, i-, sek.- und tert.Bu), besonders bevorzugt Si(OMe)₄, beispielsweise wie bei Murakami et al. beschrieben (s.o.) einsetzen.

Als Kohlenstoff-Zinnverbindungen eignen sich insbesondere Tetraalkylzinnverbindungen wie SnR₄ (R=Me, Et, i-Pr) sowie SnPh₄ und Sn(Cyclohexyl)₄ und Bu₃SnH (beispielsweise beschrieben von Benazzi et al. (s.o.).

Als Siliziumwasserstoffverbindungen eignen sich vorzugsweise Silan und Disilan (siehe Vansant et al., bereits weiter oben genannt).

Als Tetraalkylorthosilikaten kann man bevorzugt C₁-C₄-Tetraorthosilikate insbesondere Tetraethylorthosilikat ("TEOS") einsetzen.

Des weiteren kann man Tri-, Di- und Monoalkyl- und Triarylchlorsilanen wie ClSiR₃ mit R = Methyl, Ethyl, n-, i-Pr, n-, i-, sek.- und tert.-Bu, Phenyl, besonders bevorzugt Trimethylchlorsilan und Triethylchlorsilan, Disiloxane wie Hexa-(C₁-C₄-alkyl)disiloxane, bevorzugt Hexamethyldisiloxan, Diboran und Alkalimetallsilikate wie Natronwasserglas und Kieselsäuresol (letzteres beispielsweise unter der Bezeichnung LUDOX® der Fa. DuPont im Handel erhältlich), einsetzen.

Eine weitere Ausgestaltung der vorliegenden Erfindung betrifft ein Verfahren zur Herstellung der erfindungsgemäßen Molekularsiebe, indem man (a) alle oder teilweise alle Hohlräume eines Molekularsiebes mit Farbmittelmolekülen füllt und anschließend seine Porendurchmesser durch Reaktion mit einem Modifizierungsmittel verkleinert, oder (b) die Porendurchmesser eines in allen oder nur teilweise allen seinen Hohlräumen mit Farbmittelmolekülen befüllten Molekularsiebes durch Reaktion mit einem Modifizierungsmittel verkleinert.

Die Menge des einzusetzenden Modifizierungsmittels hängt in der Regel von dem gewünschten Ziel ab, d.h. wie stark beispielsweise das Ausbluten oder das Auslaugen unterbunden werden soll. Man setzt daher üblicherweise eine effektiv wirksame Menge ein, die gegebenenfalls durch Vorversuche ermittelt werden kann.

In einer bevorzugten Ausführungsform setzt man Silizium-haltige Modifizierungsmittel wie die bereits weiter oben genannten - ein und wählt beispielsweise die eingesetzte Menge so, daß der Siliziumgehalt des Farbmittel enthaltenden Molekularsiebes im Bereich von 3 bis 60, bevorzugt von 10 bis 50, besonders bevorzugt von 20 bis 40 Gew.-%, bezogen auf das Farbmittel enthaltende Molekularsieb, erhöht wird.

Die Temperatur bei der Reaktion mit dem Modifizierungsmittel hängt im allgemeinen von der Art des Modifizierungsmittels, der gewünschten Geschwindigkeit, mit der das Modifizierungsmittel aufgebracht werden soll, sowie der gewünschten Schichtdicke ab. Die Obergrenze des Temperaturbereiches wird in der Regel durch die Hitzestabilität des Farbmittels vorgegeben. Die entsprechenden Temperaturbereiche sind literaturbekannt (s.o.) und vom Fachmann, ggf. durch entsprechende Vorversuche leicht zu ermitteln. Beispielsweise wählt man bei der Verwendung von Siliciumtetrachlorid eine Temperatur im Bereich von 20 bis 300°C, bevorzugt von 100 bis 200°C, beim Einsatz von TEOS eine Temperatur im Bereich von 20 bis 400, vorzugsweise von 20 bis 250, insbesondere von 50 bis 150°C.

Der Druck bei der Reaktion mit dem Modifizierungsmittel hängt im allgemeinen von der Art des Modifizierungsmittels, der gewünschten Geschwindigkeit, mit der das Modifizierungsmittel aufgebracht werden soll, der gewünschten Schichtdicke sowie der Methode ab, wie die Aufbringung durchgeführt werden soll: in der Gas- oder der Flüssigphase.

Bei der Reaktion mit dem Modifizierungsmittel in der Flüssigphase wird im allgemeinen das Modifizierungsmittel mit dem farbmittelbeladenen Molekularsieb für einen Zeitraum im Bereich von 0,5h bis 3 Tagen, bevorzugt 0,5 bis 2 Tagen, in Kontakt gebracht, beispielsweise, indem man die Komponenten zusammenbringt, gewünschtenfalls in Gegenwart eines Lösungsmittels, und rührt.

Gewünschtenfalls kann man je nach Art des Modifizierungsmittels an die vorbeschriebene Behandlung einen Hydrolyseschritt anschließen, bevorzugt bei Modifizierungsmitteln, die erst in Gegenwart von Wasser mit dem Molekularsieb reagieren, insbesondere dann, wenn das Molekularsieb oder die flüssige Phase insgesamt für die Reaktion eine nicht ausreichende Menge an Wasser enthält.

Wählt man die Gasphase als Reaktionsmedium, dann wird in der Regel das Modifizierungsmittel mit einem üblichen Inertgas wie Stickstoff und Edelgase wie Helium, Neon und Argon als Träger über das farbmittelbeladene Molekularsieb geleitet. Gewünschtenfalls kann man auch bei der Arbeitsweise in der Gasphase einen Hydrolyseschritt, wie weiter oben bereits beschrieben, anschließen.

Gewünschtenfalls kann man das Molekularsieb vor und/oder nach der Aufbringung des Modifizierungsmittels trocknen, d.h. bis auf einen gewünschten Grad von Wasser in an sich üblicher Art und Weise befreien.

In einer besonders bevorzugten Ausführungsform führt man die Reaktion mit dem Modifizierungsmittel Siliciumtetrachlorid durch, indem man einen Inertgasstrom, vorzugsweise mit Stickstoff als Inertgas, durch Siliziumtetrachlorid leitet, das eine Temperatur im Bereich von -10 bis 120, bevorzugt von 0 bis 60, besonders bevorzugt von 15 bis 40°C aufweist. Den derart gesättigten Inertgasstrom leitet man dann über eine Probe des farbmittelbeladenen Molekularsiebes, das man auf eine Temperatur im Bereich von 20 bis 300, bevorzugt von 120 bis 180°C gebracht hat. Den Druck wählt man im allgemeinen im Bereich von üblicherweise 10 bis 300, bevorzugt von 90 bis 110 kPa. Die Reaktionszeit wählt man in der Regel im Bereich von 5 min bis 10 h, bevorzugt von 30 min bis 5 h, besonders bevorzugt von 1 bis 3 h. Die Menge des Gasflusses wählt man üblicherweise im Bereich von 0,25 bis 7,5, vorzugsweise von 0,5 bis 2,5, besonders vorzugsweise von 0,75 bis 2 l/h pro g farbmittelbeladenes Molekularsieb.

In einer weiteren bevorzugten Ausführungsform setzt man ein farbmittelbeladenes Molekularsieb ein, dessen Gehalt an Wasser größer als Null beträgt, bevorzugt dessen Gehalt an Wasser genügend groß ist, um die Reaktion des Modifizierungsmittels mit den funktionellen Gruppen des Molekularsiebes, beispielsweise den OH-Gruppen, zu beschleunigen.

Nach bisherigen Beobachtungen ist für den Erfolg der Erfindung die Wahl des Farbmittels und des Molekularsiebes nicht maßgebend.
Farbmittelbeladene Molekularsiebe sind im allgemeinen über drei Herstellungswege zugänglich:
(a) durch Behandlung einer Mischung aus einem üblicherweise kalziniertem Molekularsieb und einem Farbmittel in der Regel bei erhöhten Temperaturen. Voraussetzung ist im allgemeinen ein sterisches und elektronisches Zusammenpassen von Farbmittelmolekül und Porensystem des Molekularsiebs.

In einer besonders bevorzugten Ausführungsform setzt man leicht sublimierbare Farbmittel, insbesondere Farbstoffe, ein. Hierbei empfiehlt es sich, die Behandlung im Vakuum vorzunehmen. Vorzugsweise erhitzt man bei dieser Methode eine Mischung aus Farbstoff und Molekularsieb bei einem Druck von weniger als 100 kPa (1 bar), vorzugsweise nicht über 5 kPa (50 mbar), besonders bevorzugt nicht über 1 kPa (10 mbar) und ganz besonders bevorzugt nicht über 0,1 kPa (1 mbar), auf eine Temperatur im Bereich von 50 bis 300°C, bevorzugt von 100 bis 250°C. Die Temperatur hält man dabei üblicherweise 0,5 bis 5 Tage, vorzugsweise 1 bis 4 Tage, insbesondere 2,5 bis 3,5 Tage, aufrecht. Nach dieser Behandlung kann überschüssiger, d.h. nicht im Molekularsieb gebundener Farbstoff durch eine geeignete Behandlung, beispielsweise durch Extraktion, insbesondere mittels einer Soxhlet-Extraktion, entfernt werden.

(b) durch Synthese von Farbmitteln in Anwesenheit von Molekularsieben ("ship-in-the-bottle"). Das Prinzip dieser Methode ist beispielsweise ausführlich in Zeolites 4 (1984) S.30 beschrieben, so daß nähere Ausführungen hierzu entbehrlich sind. Nach dieser Methode werden bevorzugt die Eduktmoleküle von der Größe her so gewählt, daß sie in die Hohlräume der Molekularsiebe passen. Im Anschluß an die Synthese des Farbmittels in den Hohlräumen des Molekularsiebes wird zweckmäßig überschüssiges Edukt und außerhalb der Hohlräume gebildetes Farbmittel, insbesondere bei Farbstoffen, durch eine geeignete Behandlung, z.B. Waschen mit einem Lösungsmittel, entfernt.

(c) durch die Synthese des Molekularsiebes in Anwesenheit des Farbmittels ("Templatsynthese"). Die Templatsynthese von Molekularsieben ist ausführlich z.B. in der US 4,018,870 und der DE-A 41 26 461 beschrieben, so daß sich hierzu nähere Ausführungen erübrigen. Im allgemeinen werden bei der Templatsynthese zu einer üblichen Molekularsieb-Synthesemischung, welche beispielsweise aus einer Silicium- und gegebenenfalls einer Aluminiumquelle besteht, das Farbmittel, bevorzugt Farbstoffe, also lösliche Verbindungen, und im allgemeinen - je nach Farbmittel - ein weiteres Templat gegeben. Die Mischung wird in der Regel bei einer Temperatur im Bereich von 100 bis 250°C hydrothermal in einem Autoklaven behandelt. Nach dieser Behandlung kann man das Molekularsieb in an sich bekannter Art und Weise, beispielsweise durch Waschen oder Extraktion mit geeigneten Lösungsmitteln oder Sieben, von unumgesetzten Edukten, insbesondere dem Farbmittel, befreien.

Gewünschtenfalls kann man vor der Beladung mit dem Farbmittel die Molekularsiebe bei erhöhter Temperatur und/oder bei vermindertem Druck entwässern.

Ein Nachweis über die Einlagerung von Farbmittelmolekülen in das Molekularsieb gelingt beispielsweise mittels Röntgenpulverdiffraktometrie und über die Bestimmung der Adsorptionskapazität von z.B. Stickstoff. Üblicherweise sind die Röntgenpulverdiffraktogramme (gemessen bei 120°C) von Farbmittel beladenen Molekularsieben und solchen ohne Farbmittel sehr ähnlich, sie unterscheiden sich jedoch im allgemeinen in der exakten Lage der Beugungslinien und den Intensitäten der einzelnen Linien. Ein Vergleich von Stickstoffadsorptionsisothermen von vollständig mit Farbmittel beladenen erfindungsgemäßen Molekularsieben und solchen ohne oder nur teilweise befüllten Molekularsieben zeigt, daß das Mikroporenvolumen bei vollständig beladenen Molekularsieben gegenüber den anderen Molekularsieben verringert ist.

Als Molekularsiebe kann man kristalline Materialien mit einer Partikelgröße im Bereich von 0,5 bis 100 µm, einheitlicher Porenstruktur und Mikro- und/oder meso-Poren einsetzen wie Zeolithe und Phosphate mit Zeolith-Struktur. Bevorzugt setzt man die Molekularsiebe als Pulver ein. Je nach Anwendungszweck kann man jedoch auch kompakte Strukturen wie Granulat, Zylinder oder ähnliches verwenden.

Zeolithe sind allgemein bekannt und beispielsweise ausführlich in Chemie in unserer Zeit, 1986,4, S. 117 - 127 und Angew. Chem. 1975, 18, S. 659 - 667 beschrieben. Üblicherweise setzt man die Zeolithe in der aciden H-Form oder neutralen Alkalimetall-Form ein. In den Zeolithen können anstelle von Aluminium auch andere Elemente wie B, Ga, Fe, Cr, V, As, Sb, Bi oder Be oder deren Kombinationen in das Gitter des Zeolithen eingebaut werden. Silicium kann durch ein anderes vierwertiges Element wie Ge, Ti, Zr oder Hf teilweise ersetzt werden.

Liegen die Zeolithe auf Grund ihrer Herstellung nicht in der aciden H-Form vor, sondern beispielweise in der Alkalimetall- oder Erdalkalimetall-Form, dann kann man durch Ionenaustausch mit z.B. Ammoniumionen und anschließender Calcinierung oder durch Behandlung mit Säuren vollkommen oder partiell die gewünschte H-Form generieren.

Bevorzugte Zeolithe sind die Zeolithe A, X, Y, L, ZSM-5, ZSM-11, Zeolithe der Mordenit-Gruppe, insbesondere Mordenit, und Zeolithe vom Faujasit-Typ. Besonders bevorzugte Zeolithe sind Zeolith HY, Zeolith NaY, Zeolith H-Mordenit und Zeolith LZY-52 (von Union Carbide).

Man kann auch mesoporöse Silikate oder Metallsilikate mit MCM-41-Struktur als Molekularsieb einsetzen. Beispielhaft seien genannt: amorpher, mesoporöser MCM-41 mit einer einstellbaren Porenweite im Bereich von im allgemeinen 3 bis 10 nm (siehe beispielsweise J.of Am.Chem.Soc. 114 (1992) 10834-10843, US 5,098,684, US 5,105,051, US 5,134,242, US 5,134,243), Molekularsiebe aus der M41S-Familie wie MCM-41 mit hexagonaler Struktur, MCM-50 mit laminaler Struktur (siehe Stud.Surf.Sci.Catal. 84 (1994) 53-60), MCM-48 mit kubischer Struktur (siehe Stud.Surf.Sci.Catal. 84 (1994) 53-60), FSM-16 (siehe Stud.Surf.Sci.Catal. 84 (1994) 125-132), Metallsilikate mit verschiedenen Metallen M (siehe WO 91/11390 für M=Al, J.Chem.Soc., Chem.Commun. (1994) 147-148 für M=Ti, J.Chem.Soc., Chem.Commun. (1994) 1059-1060 für M=V, und Prep.6th Int.Symp.Sci. Bases Heterog.Cat. 1 (1994) 345-352 für M=W, Mo, Pb und Fe).

Auch ist bekannt, daß durch eine mehrfache Anwendung von Wasserdampf- und Säurebehandlung ein viel höheres Si/Al-Verhältnis zu erreichbar ist als bei einer einfachen Behandlung mit Wasser oder Säure. Solchermaßen behandelte Zeolithe (Faujasite) weisen ein mesoporöses System auf, d.h. sie enthalten Poren mit einem Durchmesser im Bereich von 2 bis 50 nm (20 bis 500 Angström). Das gesamte Volumen dieses mesoporösen Systems wird hauptsächlich von der ursprünglichen Anzahl der Al-Atome im Zeolithgerüst beeinflußt. Dabei sind im allgemeinen die auf diese Weise erzeugten Mesoporen mit der äußeren Oberfläche des Zeolithen verbunden (Inorg.Chem. 15(2) (1976) 295-297; J.Phys.Chem. 93 (1989) 3677-3683; Zeolites 14 (1994) 533-540; Microporous Materials 6 (1996) 311-320).

Man kann die poröse Struktur von Molekularsieben, insbesondere von Zeolithen, durch Stickstoffadsorption charakterisieren. Das Volumen des mikroporösen Systems (Porendurchmesser kleiner als 2 nm (20 Angström)) wird üblicherweise mit Hilfe der sog. t-plot-Gleichung ermittelt. Die Bestimmung der spezifischen Oberfläche von mikro- und mesoporösen Festkörpern erfolgt im allgemeinen nach der BET-Methode. Die mesoporöse Struktur kann man mit Hilfe des sog. BJH-Modells charakterisieren (siehe z.B. J.Am.Chem.Soc. 73 (1951) 373-380).

Phosphate mit Zeolith-Struktur - sogenannte AlPO's, SAPO's, ELSAPO's, ELAPO's, MeAPO's und ZYT's - sind beispielsweise in "Alumophosphate moleculare sieves and the periodic table" Pure & Appl. Chem. Vol 58, Nr 10 S. 1351 bis 1358 (1986), US 4,310,440 (AIPO), EP-A-103,117, US 4,440,871 (SAPO) und J 59/217,619 (ZYT) ausführlich beschrieben.

Beispielhaft genannt seien AIPO-5, AIPO-8, AIPO-9, AIPO-11, AIPO-12, AIPO-14, AIPO-21, AIPO-25, AIPO-31, AIPO-33 sowie MCM-9. Synthesen dieser Verbindungen sind in der EP-A 132 708, US-A 4,310,440 und J.Am.Chem.Soc. 104 (1982) 1146 beschrieben. Als SAPO einsetzbare Verbindungen seien genannt SAPO-5, SAPO-8, SAPO-11, SAPO-31 und SAPO-34. Die Herstellung dieser Verbindungen ist in der EP-A 103,117 und US-A 4,440,871 ausführlich beschrieben. Als weitere einsetzbare Siliciumaluminiumphosphate seien beispielhaft genannt ZYT-5, ZYT-6, ZYT-7, ZYT-9 und ZYT-12 (siehe J 59 217 619).

Des weiteren kann man Molekularsiebe mit VPI-5 Struktur (M.E. Davis et al. J.Phys.Chem. 1991, 95 S. 1380 - 1383), Cloverit (Galliumphosphat; Nature 352 (1991) S. 320 - 322) und JDF-20 (J. Chem. Soc. Chem. Commun. (1992) S. 875 - 876) einsetzen.
Die Porengröße der Molekularsiebe wählt man in einer bevorzugten Ausführungsform im Bereich von 0,4 bis 1,4 nm (4 bis 14 Å), vorzugsweise von 0,5 bis 1,2 nm (5 bis 12 Å), insbesondere von 0,5 bis 0,8 nm (5 bis 8 Å). Man kann aber auch - wie weiter oben bereits beschrieben, mesoporöse Zeolithe mit einer Porengröße im Bereich von 2 bis 50 nm einsetzen.
Als Farbmittel kann man sowohl lösliche (Farbstoffe) als auch unlösliche (Pigmente) Verbindungen einsetzen.

Aus praktischen Gründen setzt man bevorzugt Verbindungen ein, die sich sublimieren lassen, so dass eine Beladung der Molekularsiebe mit dem Farbmittel in der Gasphase vorgenommen werden kann.

Beispielhaft genannt seien Azopigmente wie Monoazo-, Disazo-, Naphthol-, Benzimidazol-, Disazokondensations-, Metallkomplex-, Isoindolinon- und Isoindolin-Pigmente, Indigo, Chinophthalon-Pigmente, Dioxazin-Pigmente sowie polycyclische Pigmente wie Chinacridon-, Phthalocyanin-, Perylen-, Perinon- und Thioindigo-, ferner Anthrachinon-Pigmente wie Aminoanthrachinon-, Hydroxyanthrachinon-, Anthrapyrimidin-, Indanthron-, Flavanthron-, Pyranthron-, Anthanthron- und Isoviolanthron-Pigmente -Pigmente und Diketopyrrolopyrrol-("DPP")-Pigmente.

Bevorzugte Farbmittel sind Anthrachinon-, DPP-, Azo- und Indigopigmente wie 1,4-Di-keto-3,6-diaryl-pyrrolo-[3,4-c]-pyrrole, besonders bevorzugt die löslichen N,N'-Dimethyl-1,4-di-keto-3,6-diaryl-pyrrolo-[3,4-c]-pyrrole wie N,N'-Dimethyl-1,4-diketo-3,6-diphenyl-pyrrolo-[3,4-c]-pyrrol.

In einer bevorzugten Ausführungsform setzt man lösliche Verbindungen ein, die man durch chemische Modifizierung von Pigmenten in an sich üblicher Art und Weise erhält, z.B. durch Einführung von üblichen polaren funktionellen Gruppen wie Sulfonsäure- oder Ammoniumgruppen.

Die genannten Farbmittel sind allgemein bekannt und in einigen Fällen kommerziell erhältlich und/oder in Analogie zu bekannten Verfahren herstellbar.

Die Höhe der Beladung des Molekularsiebes mit dem Farbmittel kann man üblicherweise durch geeignete Wahl des Molekularsiebes und/oder durch Wahl des Mengenverhältnisses von Farbmittel zu Molekularsieb steuern.

Beispielsweise beeinflußt die Art und Menge der Alkalimetallionen in einem Molekularsieb die Höhe der Beladung. So gelingt z.B. die Einlagerung von N,N'-Dimethyl-1,4-diketo-3,6-diphenyl-pyrrolo-[3,4-c]-pyrrol in den Zeolithen LiX-90 zu maximal 13, in den Zeolithen NaX-100 zu maximal 12, in den Zeolithen KX-100 zu maximal 7 Gew.-%, bezogen auf das Gewicht des entsprechenden Zeolithen. Entsprechend kann man den Zeolithen LiY-65 mit dem genannten Farbmittel zu maximal 13, mit dem Zeolithen NaY-100 zu maximal 12 und mit dem Zeolithen KY-100 zu maximal 3 Gew.-%, bezogen auf das Gewicht des entsprechenden Zeolithen, beladen.

Des weiteren kann man beispielsweise die maximale Beladung des Zeolithen NaY-100 (12 Gew.-%) mit einem Mengenverhältnis von dem genannten Farbmittel N,N'-Dimethyl-1,4-diketo-3,6-diphenyl-2,5-dihydro-pyrrolo-[3,4-c]-pyrrol zu Zeolith NaY-100 von 1 zu 3 durchführen, während man beispielsweise bei einer Beladung mit einem Mengenverhältnis von 1 zu 30 zu einer Beladung von maximal 3 Gew.-% gelangen kann.

Das Gewichtsverhältnis von Farbmittel zu Molekularsieb wählt man in der Regel im Bereich von 0,01 bis 60, bevorzugt von 0,5 bis 40, besonders bevorzugt von 0,5 bis 35 Gew.-%, bezogen auf das Gewicht des (unbeladenen) Molekularsiebes.

Die erfindungsgemäßen Molekularsiebe eignen sich vorteilhaft für viele Zwecke wie zur Einfärbung von hochmolekularen organischen Materialien wie Biopolymeren, Kunststoffen, inklusive Fasern, Gläsern, keramischen Produkten, für Zubereitungen in der dekorativen Kosmetik, zur Herstellung von Tinten, Druckfarben, Lacken, insbesondere Automobillacken, und Dispersionsfarben.

Beispiele geeigneter hochmolekularer organischer Materialien, die mit den erfindungsgemäßen Verbindungen gefärbt werden können, sind Vinylpolymere wie Polystyrol, Poly-α-methylstyrol, Poly-p-methylstyrol, Poly-p-hydroxystyrol, Poly-p-hydroxyphenylstyrol, Polymethylmethacrylat und Polyacrylamid sowie die entsprechenden Methacrylverbindungen, Polymethylmaleat, Polyacrylnitril, Polymethacrylnitril, Polyvinylchlorid, Polyvinylfluorid, Polyvinylidenchlorid, Polyvinylidenfluorid, Polyvinylacetat, Polymethylvinylether und Polybutylvinylether; von Maleinimid und/oder Maleinanhydrid abgeleitete Polymere wie Copolymere von Maleinanhydrid mit Styrol; Polyvinylpyrrolidon; ABS; ASA; Polyamide; Polyimide; Polyamidimide; Polysulfone; Polyethersulfone; Polyphenylenoxide; Polyurethane; Polyharnstoffe; Polycarbonate; Polyarylene; Polyarylensulfide; Polyepoxide; Polyolefine wie Polyethylen und Polypropylen; Polyalkadiene; Biopolymere und deren Derivate wie Cellulose, Celluloseether und -ester wie Ethylcellulose, Nitrocellulose, Celluloseacetat und Cellulosebutyrat, Stärke, Chitin, Chitosan, Gelatine, Zein; natürliche Harze; Kunstharze wie Alkydharze, Acrylharze, Phenolharze, Epoxidharze, Aminoformaldehydharze wie Harnstoff- und Melamin-Formaldehydharze; Gummi; Casein; Silikon und Silikonharze; Kautschuk, Chlorkautschuk; des weiteren Polymere, die beispielsweise als Bindemittel in Lacken verwendet werden wie Novolacke abgeleitet von C₁-C₆-Aldehyden wie Formaldehyd und Acetaldehyd und einem zweikernigen oder einkernigen, vorzugsweise einkernigen Phenol, das gewünschtenfalls mit einer oder zwei C₁-C₉-Alkylgruppen, einem oder zwei Halogenatomen oder einem Phenylring substituiert ist wie o-, m- oder p-Kresol, Xylol, p-tert.-Butylphenol, o-, m- oder p-Nonylphenol, p-Chlorphenol oder p-Phenylphenol, oder einer Verbindung mit mehr als einer phenolischen Gruppe wie Resorcin, Bis-(4-hydroxyphenyl)methan oder 2,2-Bis-(4-hydroxyphenyl)propan; sowie geeignete Mischungen der genannten Materialien.

Besonders bevorzugte hochmolekulare organische Materialien, insbesondere zur Herstellung eines Lackes, einer Druckfarbe oder Tinte, sind beispielsweise Celluloseether und -ester, wie Ethylcellulose, Nitrocellulose, Celluloseacetat und Cellulosebutyrat, natürliche Harze oder Kunstharze (Polymerisations- oder Kondensationsharze) wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, ASA, Polyphenylenoxide, Gummi, Casein, Silikon und Silikonharze sowie deren mögliche Mischungen untereinander.

Man kann auch hochmolekulare organische Materialien in gelöster Form als Filmbildner einsetzen wie Leinölfirnis, Nitrocellulose, Alkydharze, Phenolharze, Melamin- und Harnstoff/Formaldehydharze sowie Acrylharze.

Die genannten hochmolekularen organischen Verbindungen können einzeln oder in Gemischen z.B. als Granulat, plastische Massen, Schmelzen oder in Form von Lösungen, insbesondere zur Herstellung von Spinnlösungen, Lacken, Anstrichstoffen, Tinten oder Druckfarben, vorliegen.
In einer besonders bevorzugten Ausführungsform verwendet man die erfindungsgemäßen Molekularsiebe zum Massefärben von Polyvinylchlorid, Polyamiden und insbesondere Polyolefinen wie Polyethylen und Polypropylen sowie zur Herstellung von Lacken, insbesondere Automobillacken sowie Pulverlacken, Tinten, Druckfarben und Anstrichfarben.

Als Beispiele für bevorzugte Bindemittel für Lacksysteme seien Alkyd/Melaminharzlacke, Acryl/Melaminharzlacke, Celluloseacetat/Cellulosebutyratlacke und Zweikomponentenlacke auf Basis mit Polyisocyanat vernetzbarer Acrylharze genannt.

Nach bisherigen Beobachtungen kann man die erfindungsgemäßen Molekularsiebe in jeder gewünschten Menge in Abhängigkeit vom Verwendungszweck dem zu färbenden Material zugeben. Beispielsweise kann man bei hochmolekularen organischen Materialien die erfindungsgemäßen Molekularsiebe in einer Menge im Bereich von 0,2 bis 40, bevorzugt von 0,5 bis 20 Gew.-%, bezogen auf das Gesamtgewicht des pigmentierten hochmolekularen organischen Materials, einsetzen.

Die Einfärbung der hochmolekularen organischen Materialien mit den erfindungsgemäßen Molekularsieben erfolgt in der Regel dergestalt, daß man die erfindungsgemäßen Molekularsiebe, gewünschtenfalls in Form von Masterbatches, den hochmolekularen organischen Materialien unter Verwendung üblicher hierzu geeigneter Vorrichtungen wie Walzwerke, Misch- oder Mahlapparaturen zumischt. Das pigmentierte Material wird im allgemeinen hierauf nach an sich bekannten Verfahren wie Kalandrieren, Pressen, Strangpressen, Streichen, Gießen oder Spritzgießen in die gewünschte endgültige Form gebracht.

Zur Herstellung nicht starrer Formlinge oder zur Verringerung ihrer Sprödigkeit kann man den hochmolekularen Substanzen vor der Verformung sogenannte Weichmacher zusetzen. Weichmacher können beispielsweise sein: Ester der Phosphorsäure, Phthalsäure und Sebazinsäure. Die Weichmacher können vor, während oder nach dem Einfärben der hochmolekularen Substanzen mit den erfindungsgemäßen Molekularsieben zugesetzt werden.

Zur Erzielung verschiedener Farbtöne kann man die erfindungsgemäßen Molekularsiebe vorteilhaft in Abmischung mit Füllstoffen, transparenten und deckenden Weiß-, Bunt-und/oder Schwarzpigmenten sowie auch herkömmlichen Glanzpigmenten in der gewünschten Menge einsetzen.

Zur Herstellung von Lacken, Anstrichstoffen, Tinten und Druckfarben dispergiert oder löst man im allgemeinen die entsprechenden hochmolekularen organischen Substanzen wie Bindemittel, Kunstharzdispersionen etc. und die erfindungsgemäßen Molekularsiebe, gewünschtenfalls zusammen mit üblichen Zusatzstoffen wie Füllmitteln, Lackhilfsmitteln, Siccativen, Weichmachern und/oder zusätzlichen Pigmenten, in einem gemeinsamen Lösungsmittel oder Lösungsmittelgemisch. Man kann dabei so verfahren, daß man die einzelnen Komponenten für sich oder auch mehrere gemeinsam dispergiert oder löst, und erst hierauf alle Komponenten zusammenbringt, oder alles auf einmal zugibt.

Bei der Applikation im Druck kann man alle industrieüblichen Druckverfahren wie Siebdruck, Tiefdruck, Bronzierdruck, Flexodruck und Offestdruck einsetzen.

Eine Ausgestaltung der vorliegende Erfindung betrifft daher auch Materialien wie hochmolekulare organische Materialien, insbesondere Biopolymere und Kunststoffe, Gläser, keramische Produkte, Zubereitungen in der dekorativen Kosmetik, Lacke, insbesondere Automobillacke, Druckfarben, Tinten, Dispersionsfarben und Farbfilter, die die erfindungsgemässen Molekularsiebe enthalten.

Die erfindungsgemäßen Molekularsiebe zeigen gegenüber farbstoffhaltigen Molekularsieben aus dem Stand der Technik ein verbessertes Auslaug-("leaching") und Ausblutverhalten, eine verbesserte thermische Stabilität, eine verbesserte Resistenz gegenüber Lösungsmitteln und eine verbesserte Lichtstabilität. Des weiteren sind die erfindungsgemäßen Molekularsiebe chemisch resistenter. Ferner ist die Beladungshöhe des Farbmittels höher als bei vergleichbaren bekannten Verbindungen.

### Beispiele

In den nachfolgenden Beispielen werden stets ausgeheizte, d.h. durch Erhitzen entwässerte Molekularsiebe verwendet. Hierbei werden die Molekularsiebe auf 450°C mit einer Aufheizrate von 1°C/min erhitzt und bei dieser Temperatur und unter vermindertem Druck bei 100 mPa (10⁻³ mbar) 12 h entwässert.

Beispiel 1: (a) 3,03 g Zeolith HY (erhalten durch Ionenaustausch aus Zeolith NaY der Degussa AG) werden mit 1,01 g Chinizarin bei einer Temperatur von 160°C drei Tage lang bei einem vermindertem Druck von 300 mPa erhitzt. Danach wird das abgekühlte Reaktionsgemisch mit 300 ml Pyridin unter Rückfluß in einer Soxhlet-Apparatur solange extrahiert, bis der sich im Überlauf der Apparatur befindliche Extrakt farblos ist. Anschliessend extrahiert man das mit Pyridin extrahierte Molekularsieb mit 300 ml Aceton unter Rückfluß solange in einer Soxhlet-Apparatur, bis der sich im Überlauf befindliche Extrakt farblos ist. Danach entfernt man das Lösungsmittel aus dem extrahierten Molekularsieb unter einem verminderten Druck von 300 mPa und einer Temperatur von 100°C während 12 h.

b) Ein Stickstoffstrom wird bei Raumtemperatur durch ein mit Siliciumtetrachlorid gefülltes Gefäß geleitet und dabei mit diesem gesättigt. Der mit SiCl₄ gesättigte Stickstoffstrom wird dann während 3 h über 2 g des nach (a) hergestellten farbmittelbeladenen Molekularsiebes, das sich in einem Röhrenofen befindet und auf 150°C erhitzt ist, geleitet. Anschließend wird überschüssiges Siliciumtetrachlorid mit einem reinen Stickstoffstrom bei Raumtemperatur durch einstündiges Nachspülen entfernt.

Beispiel 2: (a) 3,00 g eines ultrastabilen, dealuminierten Zeolith HY (von PQ Corporation) werden mit 1,00 g Indigo bei einer Temperatur von 240°C drei Tage lang bei einem Druck von 300 mPa erhitzt. Danach wird das abgekühlte Reaktionsgemisch mit 300 ml Pyridin unter Rückfluß in einer Soxhlet-Apparatur solange extrahiert, bis der sich im Überlauf der Apparatur befindliche Extrakt farblos ist. Anschließend extrahiert man das mit Pyridin extrahierte Molekularsieb mit 300 ml Aceton unter Rückfluß solange in einer Soxhlet-Apparatur, bis der sich im Überlauf befindliche Extrakt farblos ist. Danach entfernt man das Lösungsmittel aus dem extrahierten Molekularsieb unter einem verminderten Druck von 300 mPa und einer Temperatur von 100°C während 12 h.

b) Ein Stickstoffstrom wird bei Raumtemperatur durch ein mit Siliciumtetrachlorid gefülltes Gefäß geleitet und dabei mit diesem gesättigt. Der mit SiCl₄ gesättigte Stickstoffstrom wird dann während 3 h über 2 g des nach (a) hergestellten farbmittelbeladenen Molekularsiebes, das sich in einem Röhrenofen befindet und auf 150°C erhitzt ist, geleitet. Anschließend wird überschüssiges Siliciumtetrachlorid mit einem reinen Stickstoffstrom bei Raumtemperatur durch einstündiges Nachspülen entfernt.

Beispiel 3: (a) 3,00 g Zeolith H-Mordenit (Firma CU Chemie Uetikon AG) werden mit 1,01 g Indigo bei einer Temperatur von 240°C drei Tage lang bei einem Druck von 300 mPa erhitzt. Danach wird das abgekühlte Reaktionsgemisch mit 300 ml Pyridin unter Rückfluß in einer Soxhlet-Apparatur solange extrahiert, bis der sich im Überlauf der Apparatur befindliche Extrakt farblos ist. Anschließend extrahiert man das mit Pyridin extrahierte Molekularsieb mit 300 ml Aceton unter Rückfluß solange in einer Soxhlet-Apparatur, bis der sich im Überlauf befindliche Extrakt farblos ist. Danach entfernt man das Lösungsmittel aus dem extrahierten Molekularsieb unter einem verminderten Druck von 300 mPa und einer Temperatur von 100°C während 12 h.

b) Ein Stickstoffstrom wird bei Raumtemperatur durch ein mit Siliciumtetrachlorid gefülltes Gefäß geleitet und dabei mit diesem gesättigt. Der mit SiCl₄ gesättigte Stickstoffstrom wird dann während 3 h über 2 g des nach (a) hergestellten farbmittelbeladenen Molekularsiebes, das sich in einem Röhrenofen befindet und auf 150°C erhitzt ist, geleitet. Anschließend wird überschüssiges Siliciumtetrachlorid mit einem reinen Stickstoffstrom bei Raumtemperatur durch einstündiges Nachspülen entfernt.

Beispiel 4: Beispiel 3 wird wiederholt, mit dem Unterschied, daß man als Farbmittel 0,84 g N,N'-Dimethyl-1 ,4-diketo-3,6-diphenyl-pyrrolo-[3,4-c]-pyrrol (hergestellt gemäß Beispiel 1 aus der US 4,585,878; im folgenden als "DPP1" bezeichnet) und 3,01 g Zeolith NaY (von Degussa AG) einsetzt. Ferner wird das Lösungsmittel während 12 h entfernt und die Temperatur bei der Beladung des Zeolithen mit DPP1 beträgt 150°C.

Beispiel 5: Beispiel 4 wird wiederholt, mit dem Unterschied, daß man anstelle von Zeolith NaY 7,01 g des Zeolithen H-Mordenit (von CU Chemie Uetikon AG) und 2,33 g DPP1 einsetzt.

Beispiel 6: Zum Testen des Verhaltens der modifizierten, farbmittelbeladenen Molekularsiebe gegenüber Auslaugen ("leaching") werden je 0,2 g der erfindungsgemäß hergestellten Molekularsiebe der Beispiele 1 bis 5 jeweils mit je 2 ml N-Methylpyrrolidon (NMP), Aceton und Pyridin als Lösungsmittel (siehe untenstehende Tabelle) überschichtet. Die so hergestellten Proben werden bei Raumtemperatur sechs Monate stehengelassen. Danach wird in den Lösungsmitteln der Proben keine Färbung beobachtet. Die gefundenen Ergebnisse sind in untenstehender Tabelle zusammengefaßt.

**Tabelle:**

| Übersicht über das "Leaching-Verhalten" der modifizierten, farbmittelbeladenen Molekularsiebe | | | | | |
|---|---|---|---|---|---|
| Beispiel | Farbmittel | Zeolith | Färbung in | | |
| | | | Aceton | Pyridin | NMP |
| 1 | Chinizarin | HY | - | - | - |
| 2 | Indigo | HY | - | - | - |
| 3 | Indigo | H-Mordenit | - | - | - |
| 4 | N,N-Dimethyl-DPP | NaY | - | - | - |
| 5 | N,N-Dimethyl-DPP | H-Mordenit | - | - | - |

Beispiel 7: (a) Das in Beispiel 4 hergestellte Molekularsieb wird unter Luft mit einer Heizrate von 2 K/min mittels DSC auf seine thermische Stabilität untersucht. Als Referenz dient Aluminiumoxid. Der Masseverlust ist bei 573°C abgeschlossen. (b) Der Versuch wird mit einem Molekularsieb durchgeführt, das analog zu dem aus Beispiel 4 hergestellt wird, mit dem Unterschied, dass man keine Modifizierung mit Siliciumtetrachlorid vornimmt. Bei diesem unmodifizierten Molekularsieb ist der Masseverlust bereits bei 526°C abgeschlossen.

Beispiel 8: 0,2 g des in Beispiel 3 hergestellten Pigments werden mit 13,3 g Polyvinylchlorid (Evipol®SH 7020 von EVC GmbH) und 7,3 ml einer Stabilisatormischung aus 92,21 Gew.-% DIDP Vestinol® der Hüls Chemie, 4,19 Gew.-% Rheoplast®39 von Ciba-Geigy und 3,6 Gew.-% IRGASTAB®BZ561 von Ciba-Geigy vermischt und auf einem Walzenstuhl während 15 Minuten bei einer Temperatur von 160°C zu einer dünnen Folie verarbeitet. Die so erzeugte farbige Folie ist migrationsbeständig, d.h. es ist kein Ausbluten des erfindungsgemäßen Molekularsiebes auf eine nicht gefärbte Folie zu beobachten.

## Patentansprüche

1. Molekularsieb, enthaltend in allen oder nur teilweise allen seinen Hohlräumen Farbmittelmoleküle sowie ein mit ihm kovalent verbundenes, seine Porendurchmesser verkleinerndes, Modifizierungsmittel.

2. Molekularsieb gemäß Anspruch 1, dadurch gekennzeichnet, daß man als Modifizierungsmittel mindestens eine Verbindung, ausgewählt aus der Gruppe, bestehend aus Metallhalogeniden, Siliconalkoxiden, Kohlenstoff-Zinnverbindungen, Silicium-Wasserstoffverbindungen, Tetraalkylorthosilikaten, Monoalkyl-, Dialkyl-, Trialkyl- und Triarylchlorsilanen, Disiloxanen, Diboran, Silikatsolen und -kolloiden sowie halogenierten Polysiloxanen, einsetzt.

3. Verfahren zur Herstellung eines Molekularsiebes gemäß den Ansprüchen 1 und 2, dadurch gekennzeichnet, daß man (a) alle oder teilweise alle Hohlräume eines Molekularsiebes mit Farbmittelmolekülen füllt und anschließend seine Porendurchmesser durch Reaktion mit einem Modifizierungsmittel verkleinert, oder (b) die Porendurchmesser eines in allen oder nur teilweise allen seinen Hohlräumen mit Farbmittelmolekülen befüllten Molekularsiebes durch Reaktion mit einem Modifizierungsmittel verkleinert.

4. Verwendung des Molekularsiebes gemäß den Ansprüchen 1 und 2 und hergestellt gemäß Anspruch 3 als Pigment zur Einfärbung von hochmolekularen organischen Materialien, bevorzugt Biopolymeren und Kunststoffen, Gläsern, keramischen Produkten, für Zubereitungen in der dekorativen Kosmetik, zur Herstellung von Lacken, bevorzugt Automobillacken, Druckfarben, Tinten, Dispersionsfarben und Farbfiltern.

5. Molekularsiebe enthaltende Materialien gemäss der Verwendung nach Anspruch 4.

## Claims

1. A molecular sieve all or only some of whose cavities contain colourant molecules and also a modifier which is covalently bound to said molecular sieve and which reduces its pore diameter.

2. A molecular sieve according to claim 1, wherein the modifier used is at least one compound selected from the group consisting of metal halides, silicone alkoxides, carbon/tin compounds, silicium/hydrogen compounds, tetraalkyl orthosilicates, monoalkyl-, dialkyl-, trialkyl- and triarylchlorosilanes, disiloxanes, diborane, silicate sols and silicate colloids and also halogenated polysiloxanes.

3. A process for the preparation of a molecular sieve according to claims 1 and 2, which comprises (a) filling all or some of the cavities of a molecular sieve with colourant molecules and subsequently reducing its pore diameter by reaction with a modifier, or (b) reducing the pore diameter of a molecular sieve filled in all or only some of its cavities with colourant molecules by reaction with a modifier.

4. Use of the molecular sieve according to claims 1 and 2 and prepared according to claim 3 as pigment for colouring high molecular weight organic materials, preferably bipolymers and plastics, glasses, ceramic products, for formulations of decorative cosmetics, for the preparation of coating materials, preferably automotive coatings, printing inks, graphics inks, emulsion paints and colour filters.

5. A material comprising a molecular sieve in accordance with the use according to claim 4.

## Revendications

1. Tamis moléculaire contenant des molécules de matière colorante dans tous ou dans une partie de tous ses espaces creux, ainsi qu'un agent de modification réduisant le diamètre de ses pores, lié à celui-ci par une liaison covalente.

2. Tamis moléculaire selon la revendication 1, caractérisé en ce qu'on utilise comme agent de modification au moins un composé pris dans le groupe comportant des halogénures métalliques, des alkoxydes de silicium, des composés carbonés d'étain, des composés hydrogénés de silicium, des ortho-silicates de tétraalkyle, des monoalkyl-, dialkyl-, trialkyl- et triarylchlorosilanes, des disiloxanes, le diborane, des sols et colloïdes de silicates ainsi que des polysiloxanes halogénés.

3. Procédé pour la préparation d'un tamis moléculaire selon les revendications 1 et 2, caractérisé en ce que (a) on remplit tous ou une partie de tous les espaces creux d'un tamis moléculaire de molécules de matière colorante et ensuite on réduit le diamètre de ses pores par réaction avec un agent de modification, ou (b) on réduit les diamètres de pores d'un tamis moléculaire rempli dans tous ou dans une partie de tous ses espaces creux de molécules de matière colorante par réaction avec un agent de modification.

4. Utilisation du tamis moléculaire selon les revendications 1 et 2 préparé selon la revendication 3 comme pigment pour la pigmentation de matières organiques de haut poids moléculaire, de préférence de biopolymères et de matières plastiques, de verres, de produits de céramique, de préparations de maquillage en cosmétique, de préparations de vernis, en particulier de vernis automobile, d'encres d'imprimerie, d'encres à écrire, de peintures de dispersion et de filtres colorés.

5. Tamis moléculaires contenant des matières conformément à l'utilisation selon la revendication 4.
